# FASCICULE DE BREVET EUROPEEN

(11) **EP 0 854 703 B1**
(45) Date de publication et mention de la délivrance du brevet: **02.01.2002**
(21) Numéro de dépôt: 96931856.7
(22) Date de dépôt: 16.09.1996
(51) Int. Cl.: A61K 7/48, A61K 7/06

(54) **COMPOSITION POUR LE TRAITEMENT DES MATIERES KERATINIQUES COMPRENANT AU MOINS UN POLYMERE SILICONE GREFFE ET AU MOINS UN POLYMERE OU COPOLYMERE EPPAISSISSANT DE (METH)ACRYLAMIDE OU D'UN DERIVE DE (METH)ACRYLAMIDE ET SES APPLICATIONS**
MITTEL ZUR BEHANDLUNG VON KERATINMATERIAL, DAS MINDESTENS EIN SILIKON GEPROPTES POLYMER UND MINDENSTENS EIN VERDICKENDES POLYMER ODER COPOLYMER VON (METH) ACRYLAMID ODER EINEM (METH) ACRYLAMIDDERIVAT ENTHALT, SOWIE DESSEN VERWENDUNGEN
COMPOSITION FOR TREATING KERATINOUS MATERIAL, INCLUDING AT LEAST ONE SILICONE-GRAFTED POLYMER AND AT LEAST ONE THICKENING POLYMER OR COPOLYMER OF (METH)ACRYLAMIDE OR A (METH)ACRYLAMIDE DERIVATIVE, AND USES THEREOF

(30) Priorité: 29.09.1995 FR 9511485
(43) Date de publication de la demande: 29.07.1998
(73) Titulaire: L'OREAL, 75008 Paris (FR)
(72) Inventeur: CAUWET-MARTIN, Danièle, 75011 Paris (FR); DUBIEF, Claude, 78150 Le Chesnay (FR); DUPUIS, Christine, 75018 Paris (FR)
(74) Mandataire: Le Blainvaux Bellegarde, Françoise
(86) Numéro de dépôt international: FR9601438
(87) Numéro de publication internationale: WO9712595

(56) Documents cités:
- EP-A- 0 412 704
- EP-A- 0 524 612
- EP-A- 0 636 361
- WO-A-91/15186
- WO-A-95/00108
- FR-A- 2 709 955

## Description

La présente invention a trait à une composition cosmétique ou dermatologique pour le traitement des matières kératiniques, en particulier des cheveux humains, comprenant au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale et au moins un polymère ou copolymère épaississant de (méth)acrylamide ou d'un dérivé de (méth) acrylamide ainsi que ses applications.

Les polymères du type polymère siliconé greffé comprenant une portion polysiloxane et une portion constituée d'une chaîne organique non-siliconée, l'une des deux portions constituant la chaîne principale du polymère l'autre étant greffée sur ladite chaîne principale sont connus pour leurs propriétés coiffantes. Ils sont particulièrement intéressants en cosmétique capillaire du fait qu'ils apportent de la tenue aux cheveux. Leurs propriétés cosmétiques après application sur les cheveux sont néanmoins insuffisantes. On constate que les cheveux présentent après application de ces polymères, un toucher rêche et crissant résultant d'une répartition discontinue du polymère le long des fibres des cheveux.

Le document WO-A-95 00108 décrit notamment les polymères silicones greffés comprenant une portion constituée d'une chaîne organique non-siliconée surlaquelle est greffée une portion polysiloxane.

La demanderesse a trouvé de façon surprenante que l'utilisation d'un polymère ou copolymère épaississant de (méth)acrylamide ou d'un dérivé de (méth)acrylamide, réticulé ou non-réticulé dans des compositions capillaires contenant un polymère du type polymère siliconé greffé comprenant une portion polysiloxane et une portion constituée d'une chaîne organique non-siliconée, l'une des deux portions constituant la chaîne principale du polymère l'autre étant greffée sur la dite chaîne principale, permettait d'améliorer, à l'application, le dépôt du polymère siliconé greffé le long des fibres kératiniques et d'améliorer leurs propriétés cosmétiques notamment au niveau du toucher tout en conservant les propriétés coiffantes du polymère siliconé greffé.

La composition selon l'invention est donc essentiellement caractérisée par le fait qu'elle contient dans un milieu cosmétiquement ou dermatologiquement acceptable au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale et au moins un polymère ou copolymère épaississant de (méth)acrylamide ou d'un dérivé de (méth)acrylamide, réticulé ou non-réticulé.

Les polymères siliconés greffés selon l'invention sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.

Dans ce qui suit, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Dans ce qui suit, on entend désigner par «macromère polysiloxane», en conformité avec l'acceptation générale, tout monomère contenant dans sa structure une chaîne polymère du type polysiloxane.

Les polymères à squelette organique non-siliconé greffé par des monomères contenant un polysiloxane conformes à la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisée sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0 à 98% en poids d'au moins un monomère(A) lipophile de faible polarité lipophile à insaturation éthylénique, polymérisable par voie radicalaire ;
b) de 0 à 98% en poids d'au moins un monomère (B) hydrophile polaire à insaturation éthylénique, copolymérisable avec le ou les monomères du type (A)
c) de 0,01 à 50 % en poids d'au moins un macromère polysiloxane (C) de formule générale :

   X(Y)ₙSi(R)₃₋ₘZₘ (I)

   où :
   X désigne un groupe vinylique copolymérisable avec les monomères (A) et (B) ;
   Y désigne un groupe de liaison divalent ;
   R désigne un hydrogène, un alkyle ou un alcoxy en C₁-C₆, un aryle C₆-C₁₂;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,693,935, US 4,728,571 et US 4,972,037 et les demandes de brevet EP-A-0412704, EP-A-0412707, EP-A-0640105. Ils ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de omégahydrydofluroalcanol ; les esters d'acide acrylique ou méthacrylique et de fluoroalkylsulfoamido-alcool; les esters d'acide acrylique ou méthacrylique et d'alcool fluoroalkylique ; les esters d'acide acrylique ou méthacrylique et de fluroéther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perflurooctane sulfonamido)-éthylacrylate ; le 2-(N-butylperflurooctane sulfonamido)-éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quatemisé, le (méth)acrylamide, le N-t-butylacrylamide, l'acide maleique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (II) : 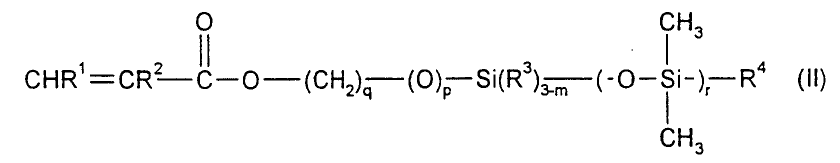 dans laquelle :
R¹ est hydrogène ou -COOH (de préférence hydrogène) ;
R² est hydrogène, méthyle ou -CH₂COOH (de préférence méthyle) ;
R³ est alkyle, alcoxy ou alkylamino en C₁-C₆, aryle en C₆-C₁₂ ou hydroxyle (de préférence méthyle) ;
R⁴ est alkyle, alcoxy ou alkylamino en C₁-C₆, aryle en C₆-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1) ;

On utilise plus particulièrement les macromères polysiloxanes de formule : 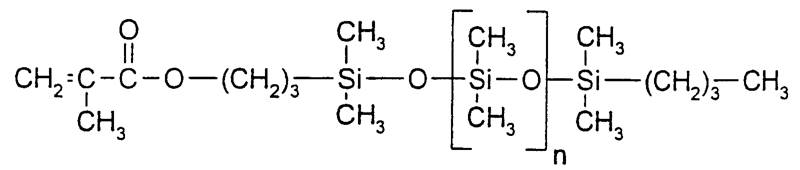 avec n étant un nombre allant de 5 à 700.

Un mode particulier de réalisation de l'invention consiste à utiliser un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 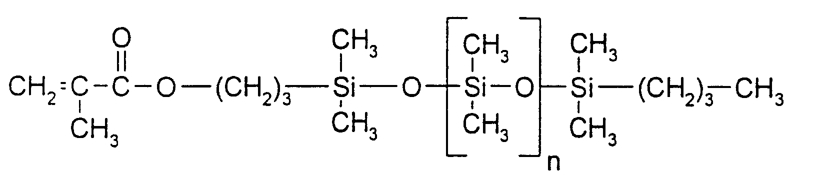 avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 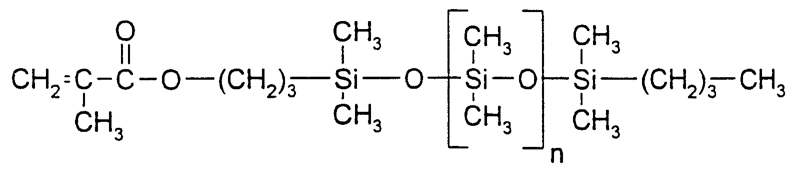 avec n étant un nombre allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, -alkylstyrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (III):

T-(CH₂)ₛ-Si-[-(OSiR⁵R⁶)ₜ-R⁷]_{y} (III)

dans laquelle T est choisi dans le groupe constitué par NH2, NHR', une fonction époxy, OH, SH ; R⁵, R⁶, R⁷ et R', indépendamment, désignent un alkyle en C₁-C₆, phenyle, benzyle, ou alkylphényle en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9.000 à 40.000.

Selon la présente invention, le ou les polymères siliconés greffés, à squelette polysiloxanique greffé par des monomères organiques non-siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo) polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (IV) suivant : 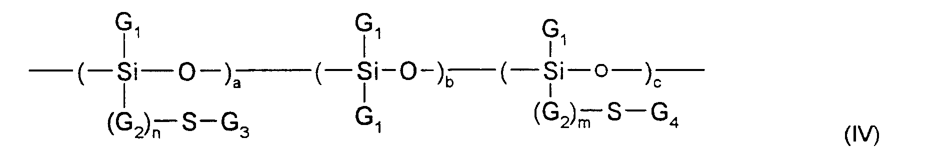 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes:
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

Les polymères greffés siliconés de l'invention sont utilisés de préférence en une quantité allant de 0,01 à 20% en poids du poids total de la composition. Plus préférentiellement, cette quantité varie de 0,1 à 15% en poids et encore plus préférentiellement de 0,5 à 10% en poids.

On peut citer comme polymères ou copolymères épaississants de (méth)acrylamide ou de dérivé de (méth)acrylamide préférentiels convenant pour les compositions de l'invention, les polymères suivants :
(i) les copolymères réticulés d'acide 2-acrylamido-2-méthyl-propane sulfonique partiellement ou totalement neutralisés (par une base telle que la soude, de la potasse ou une amine) et d'acrylamide tels que le produit décrit dans l'exemple 1 du document EP-A-503 853 ;
(ii) les copolymères d'acrylate d'ammonium et de (méth)acrylamide, réticulés ou non-réticulés, tels que le produit vendu sous le nom BOZEPOL® C NOUVEAU ou le produit PAS 5193® vendus par la société HOECHST (ils sont décrits et préparés dans les documents FR 2416723, USP2798053 et USP 2923692) ;
(iii) les copolymères de diméthylamino-éthylméthacrylate quaternisé par le chlorure de méthyle et de (méth)acrylamide , réticulés ou non-réticulés, tels que le produit SALCARE® SC92 vendu par ALLIED COLLOIDS ou le produit PAS 5194® vendu par HOECHST (ils sont décrits dans le document EP-A-395282) ;
(iv) leurs mélanges.

Les polymères ou copolymères de (méth)acrylamide ou d'un dérivé de (méth)àcrylamide selon l'invention, sont utilisés en une quantité allant de 0,01 à 20% en poids du poids total de la composition. Plus préférentiellement, cette quantité varie de 0,1 à 15% en poids et encore plus préférentiellement de 0,5 à 10% en poids.
Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit mileu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement non-thérapeutique des matières kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLES

### EXEMPLE 1 Après-shampooing

- Polymère siliconé greffé de formule (IV)
   de structure polyméthyl/méthylsiloxane
   groupements propyl thio-3 polyméthacrylate d'isobutyle 2 g en MA
- Copolymère réticulé acrylamide/chlorure de méthacrylate
   de triméthyléthylammonium 42/58 en dispersion à 50 %
   dans l'huile vendu sous le nom SALCARE® SC 92
   par ALLIED COLLOIDS 2 g en MA
- Parfum, conservateur qs
- Eau qsppH ajusté à 7 par NaOH 100 g

### EXEMPLE 2 Après-shampooing

- Polymère siliconé greffé de formule (IV) de structure
   polyméthyl/méthylsiloxane à groupements propyl
   thio-3 acide polyméthacrylique et groupements
   propyl thio-3 polyméthacrylate de méthyle 1 g en MA
- Copolymère réticulé acrylamidelacide acrylamido
   2- méthylpropane sulfonique sous forme de sel de
   sodium en émulsion inverse à 40% dans un mélange
   isoparaffine/eau tel que le produit décrit dans l'exemple 1
   du document EP-A-503 853 1 g en MA
- Polydiméthylsiloxane 350 est 1 g
- Parfum, conservateur qs pH ajusté à 6 par NaOH
- Eau qsp 100 g

## Revendications

1. Composition cosmétique ou dermatologique destinée au traitement des matières kératiniques, **caractérisée par le fait qu'**elle contient dans un milieu cosmétiquement ou dermatologiquement acceptable au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale et au moins un polymère ou copolymère épaississant de (méth)acrylamide ou d'un dérivé de (méth)acrylamide, réticulé ou non-réticulé.

2. Composition selon la revendication 1, **caractérisée par le fait que** le polymère siliconé greffé est choisi dans le groupe constitué par les polymères à squelette organique non-siliconé greffé par des monomères contenant un polysiloxane, les polymères à squelette polysiloxanique greffé par des monomères organiques non-siliconés et leurs mélanges.

3. Composition selon la revendication 1 ou 2, **caractérisée par le fait qu'**elle contient au moins un polymère siliconé greffé, à squelette organique non-siliconé, constitué d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

4. Composition selon la revendication 3, **caractérisée par le fait que** les monomères organiques non-siliconés constituant la chaîne principale du polymère siliconé greffé sont choisis dans le groupe constitué par des monomères à insaturation éthylènique polymérisables par voie radicalaire, des monomères polymérisables par polycondensation, des monomères à ouverture de cycle.

5. Composition selon l'une quelconque des revendications 1 à 4, **caractérisée par le fait qu'**elle contient au moins un copolymère greffé siliconé comprenant :
a) de 0 à 98% en poids d'au moins un monomère (A) lipophile de faible polarité à insaturation éthylénique de faible polarité, polymérisable par voie radicalaire ;
b) de 0 à 98% en poids d'au moins un monomère (B) hydrophile polaire à insaturation éthylénique, copolymérisable avec le ou les monomères du type (A)
c) de 0,01 à 50 % en poids d'au moins un macromère polysiloxane (C) de formule générale :
X(Y)ₙSi(R)₃₋ₘ Zₘ (I)
où :
X désigne un groupe vinylique copolymérisable avec les monomères (A) et (B) ;
Y désigne un groupe de liaison divalent ;
R désigne un hydrogène, un alkyle ou un alcoxy en C₁-C₆, un aryle C₆-C₁₂ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

6. Composition selon la revendication 5, **caractérisée par le fait que** les monomères lipophiles (A) sont choisis dans le groupe constitué par les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool ; les esters d'acide acrylique ou méthacrylique et d'alcools fluoroalkylique ; les esters d'acide acrylique ou méthacrylique et de fluoroéthers d'alcools ; ou leurs mélanges.

7. Composition selon la revendication 6, **caractérisée par le fait que** les monomères lipophiles (A) sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluorooctane sulfonamido)-éthylacrylate; le 2-(butylperfluorooctane sulfonamido)-éthylacrylate.

8. Composition selon l'une quelconque des revendications 5 à 7, **caractérisée par le fait que** les monomères polaires (B) sont choisis dans le groupe constitué par l'acide acrylique, l'acide méthacrylique, le N,N-diméthylacrylamide, le méthacrylate de diméthylaminoéthyle, le méthacrylate de diméthylaminoéthyle quatemisé, le (méth) acrylamide, le N-t-butylacrylamide, l'acide maleique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges.

9. Composition selon la revendication 8, **caractérisée par le fait que** les monomères polaires (B) sont choisis dans le groupe constitué par l'acide acrylique, le N,N-diméthylacrylamide, le méthacrylate de diméthylaminoéthyle, le méthacrylate de diméthylaminoéthyle quatemisé, la vinylpyrrolidone, et leurs mélanges.

10. Composition selon l'une quelconque des revendications 5 à 9, **caractérisée par le fait que** le macromère polysiloxane (C) correspond à la formule générale suivante (II) : 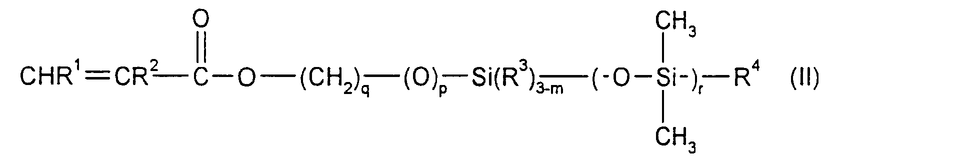 dans laquelle :
R¹ est hydrogène ou -COOH ;
R² est hydrogène, méthyle ou -CH₂COOH ;
R³ est alkyle, alcoxy ou alkylamino en C₁-C₆, aryle en C₆-C₁₂ ou hydroxyle ;
R⁴ est alkyle, alcoxy ou alkylamino en C₁-C₆, aryle en C₆-C₁₂ ou hydroxyle ;
q est un entier de 2 à 6 ;
p est 0 ou 1 ;
r est un nombre entier de 5 à 700 ;
m est un entier allant de 1 à 3.

11. Composition selon l'une quelconque des revendications 5 à 10, **caractérisée par le fait que** le macromère polysiloxane (C) correspond à la formule générale suivante : 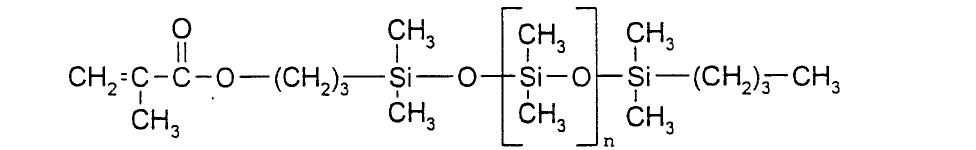 avec n étant un nombre allant de 5 à 700.

12. Composition selon l'une quelconque des revendications 1 à 11, **caractérisée par le fait qu'**elle contient au moins un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 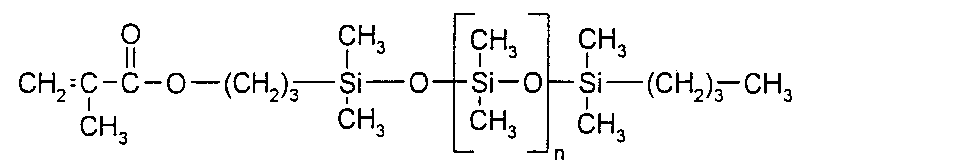 avec n étant un nombre allant de 5 à 700 ; les pourcentages en poids étant calculés par rapport au poids total des monomères.

13. Composition selon l'une quelconque des revendications 1 à 11, **caractérisée par le fait qu'**elle contient au moins un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 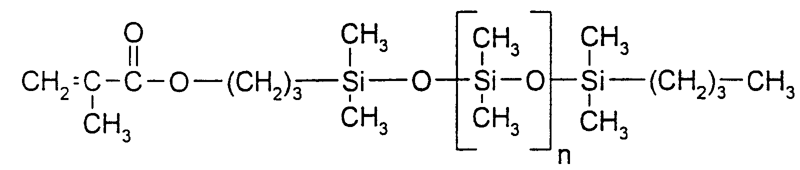 avec n étant un nombre allant de 5 à 700 ; les pourcentages en poids étant calculés par rapport au poids total des monomères.

14. Composition selon l'une quelconque des revendications 5 à 13, **caractérisée par le fait que** le polymère à squelette organique non-siliconé greffé par des monomères contenant un polysiloxane a un poids moléculaire moyen en nombre allant de 10.000 à 2.000.000 et une température de transition vitreuse Tg ou une température de fusion cristalline Tm d'au moins -20°C.

15. Composition selon l'une quelconque des revendications 1 à 4, **caractérisée par le fait qu'**elle contient au moins un polymère à squelette organique non-siliconé greffé par des monomères contenant un polysiloxane, susceptible d'être obtenu par extrusion réactive d'un macromère polysiloxane ayant une fonction réactive terminale sur un polymère du type polyoléfine comportant des groupes réactifs susceptibles de réagir avec la fonction réactive terminale du macromère polysiloxane pour former une liaison covalente permettant le greffage de la silicone sur la chaîne principale de la polyoléfine.

16. Composition selon la revendication 15, **caractérisée par le fait que** la polyoléfine réactive est choisie dans le groupe constitué par les polyéthylènes ou les polymères de monomères dérivés de l'éthylène comportant des fonctions réactives susceptibles de réagir avec la fonction terminale du macromère polysiloxane.

17. Composition selon la revendication 15 ou 16, **caractérisée par le fait que** la polyoléfine réactive est choisie dans le groupe constitué par les copolymères d'éthylène ou de dérivés d'éthylène et de monomères choisis parmi ceux comportant une fonction carboxylique ; ceux comportant une fonction anhydride d'acide ; ceux comportant une fonction chlorure d'acide ; ceux comportant une fonction ester ; ceux comportant une fonction isocyanate.

18. Composition selon l'une quelconque des revendications 15 à 17, **caractérisée par le fait que** le macromère polysiloxane est un polysiloxane comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires.

19. Composition selon l'une quelconque des revendications 15 à 18, **caractérisée par le fait que** le macromère polysiloxane est un polysiloxane répondant à la formule générale (III):
T-(CH₂)ₛ-Si-[-(OSiR⁵R⁶)ₜ-R⁷]_{y} (III)
dans laquelle T est choisi dans le groupe constitué par NH2, NHR', une fonction époxy, OH, SH ; R⁵, R⁶, R⁷ et R', indépendamment, désignent un alkyle en C₁-C₆, phényle, benzyle, ou alkylphényle en C₆-C₁₂, hydrogène ; s est un nombre de 2 à 100 ; t est un nombre de 0 à 1000 et y est un nombre de 1 à 3.

20. Composition selon la revendication 1 ou 2, **caractérisée par le fait qu'**elle contient au moins un polymère siliconé greffé, à squelette polysiloxanique greffé par des monomères organiques non-siliconés, comprenant une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

21. Composition selon la revendication 20, **caractérisée par le fait que** le polymère à squelette polysiloxanique greffé par des monomères organiques non-siliconés est susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part un polysiloxane présentant dans sa chaîne au moins un groupement fonctionnel capable de venir réagir sur lesdites insaturations éthyléniques desdits monomères non-siliconés.

22. Composition selon la revendication 21, **caractérisée par le fait que** le monomère organique anionique à insaturation éthylénique est choisi, seul ou sous forme de mélange de monomères, parmi les acides carboxyliques insaturés, linéaires ou ramifiés.

23. Composition selon la revendication 22, **caractérisée par le fait que** le monomères organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

24. Composition selon la revendication 22, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en C₁-C₁₈.

25. Composition selon la revendication 24, **caractérisée par le fait que** le monomère organique hydrophobe à insaturation éthylénique est choisi, seul ou en mélanges de monomères dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle.

26. Composition selon l'une quelconque des revendications 20 à 25, **caractérisée par le fait que** le polymère siliconé greffé comprend sur la chaîne silicone principale, au moins un groupement organique à caractère anionique obtenu par l'(homo)-polymérisation radicalaire d'au moins un monomère anionique de type acide carboxylique insaturé, partiellement ou totalement neutralisé sous la forme d'un sel.

27. Composition selon l'une quelconque des revendications 20 à 26, **caractérisée par le fait que** le polymère siliconé greffé est choisi parmi les polymères siliconés comportant dans leur structure le motif de formule (IV) suivant : 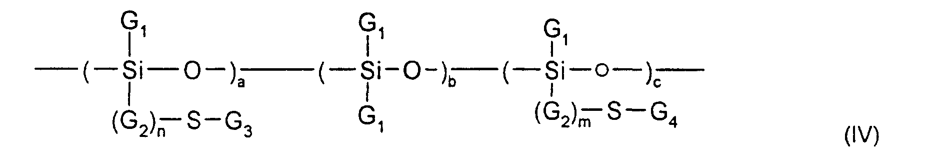 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

28. Composition selon la revendication 27, **caractérisée par le fait que** le motif de formule (IV) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle (C₁-C₁₀).

29. Composition selon la revendication 27 ou 28, **caractérisée par le fait que** le motif de formule (IV) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobuyle ou de méthyle.

30. Composition selon l'une quelconque des revendications 20 à 29, **caractérisée par le fait que** la masse moléculaire en nombre du polymère à squelette polysiloxanique greffé par des monomères organiques non-siliconés varie de 10 000 à 1 000 000 environ, et encore plus préférentiellement de 10 000 à 100 000 environ.

31. Composition selon l'une quelconque des revendications 1 à 30, **caractérisée par le fait que** le ou les polymères siliconés greffés sont utilisés en une quantité allant de 0,01 à 20% en poids par rapport au poids total de la composition et de préférence de 0,1 à 15% en poids et encore plus préférentiellement de 0,5 à 10% en poids.

32. Composition selon l'une quelconque des revendications 1 à 31, **caractérisée par le fait que** le polymère ou copolymère épaississant de (méth)acrylamide ou d'un dérivé de (méth)acrylamide est choisi dans le groupe constitué par:
(i) les copolymères réticulés d'acide 2-acrylamido-2-méthyl-propane sulfonique, partiellement ou totalement neutralisés et d'acrylamide,
(ii) les copolymères d'acrylate d'ammonium et de (méth)acrylamide, réticulés ou non-réticulés ;
(iii) les copolymères de diméthylamino-éthylméthacrylate quaternisé par le chlorure de méthyle et de (méth)acrylamide, réticulés ou non-réticulés ;
(iv) leurs mélanges.

33. Composition selon l'une quelconque des revendications 1 à 32, **caractérisée par le fait que** le ou les polymères ou copolymères épaississants de (méth)acrylamide ou d'un dérivé de (méth)acrylamide sont utilisés en une quantité allant de 0,01 à 20% en poids du poids total de la composition et plus préférentiellement de 0,1 à 15% en poids et encore plus préférentiellement de 0,5 à 10% en poids.

34. Composition selon l'une quelconque des revendications 1 à 33, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

35. Composition selon l'une quelconque des revendications 1 à 34, **caractérisée par le fait que** le milieu cosmétiquement ou dermatologiquement acceptable est constitué par de l'eau ou un mélange d'eau et d'au moins un solvant cosmétiquement acceptable.

36. Composition selon la revendication 35, **caractérisée par le fait que** les solvants cosmétiquement acceptables sont choisis dans le groupe constitué par les monoalcools, les polyalcools, les éthers de glycol, les esters d'acides gras et leurs mélanges.

37. Composition selon l'une quelconque des revendications 1 à 36, **caractérisée par le fait que** le polymère siliconé greffé est dissous dans le milieu cosmétiquement ou dermatologiquement acceptable ou utilisé sous forme de dispersion aqueuse de particules .

38. Composition selon l'une quelconque des revendications 1 à 36, **caractérisée par le fait que** les matières kératiniques sont des cheveux humains.

39. Composition selon l'une quelconque des revendications 1 à 38, **caractérisée par le fait qu'**elle se présente sous forma de gel, de lait, de crème, de lotion plus ou moins épaissie ou de mousse.

40. Composition selon l'une quelconque des revendications 1 à 39, **caractérisée par le fait qu'**elle est un produit de coiffage.

41. Composition selon l'une quelconque des revendications 1 à 40, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

42. Composition selon l'une quelconque des revendications 1 à 41, **caractérisée par le fait qu'**elle est conditionnée sous forme de vaporisateur, de flacon pompe ou bien dans un récipient aérosol en vue d'obtenir un spray, une laque ou une mousse.

43. Procédé non-thérapeutique de traitement des matières kératiniques en particulier des cheveux humains, **caractérisé par le fait qu'**il consiste à appliquer sur lesdites matières une composition telle que définie selon l'une quelconque des revendications 1 à 42 puis à effectuer éventuellement un rinçage à l'eau.

## Patentansprüche

1. Kosmetische oder dermatologische Zusammensetzung, die zur Behandlung von Keratinsubstanzen vorgesehen ist, **dadurch gekennzeichnet, daß** sie in einem kosmetisch oder dermatologisch akzeptablen Medium mindestens ein gepfropftes Siliconpolymer mit einem Polysiloxanbereich und einem Bereich, der aus einer nicht siliconhaltigen, organischen Kette besteht, wobei ein Bereich die Hauptkette des Polymers bildet und der andere auf die Hauptkette gepfropft ist, und mindestens ein vernetztes oder nicht vernetztes verdickendes Polymer oder Copolymer von (Meth)acrylamid oder eines (Meth)acrylamidderivats enthält.

2. Zusammensetzung nach Anspruch 1, **dadurch gekennzeichnet, daß** das gepfropfte Siliconpolymer unter den Polymeren, die ein nicht siliconhaltiges, organisches Grundgerüst aufweisen, auf das polysiloxanhaltige Monomere gepfropft sind, Polymeren, die ein Polysiloxangerüst aufweisen, auf das nicht siliconhaltige, organische Monomere gepfropft sind, und deren Gemischen ausgewählt ist.

3. Zusammensetzung nach Anspruch 1 oder 2, **dadurch gekennzeichnet, daß** sie mindestens ein gepfropftes Siliconpolymer mit einem nicht siliconhaltigen, organischen Grundgerüst enthält, das aus einer organischen Hauptkette besteht, die aus organischen Monomeren gebildet wird, die kein Silicon enthalten, auf die in der Kette sowie gegebenenfalls an mindestens einem ihrer Enden ein Polysiloxanmakromer gepfropft ist.

4. Zusammensetzung nach Anspruch 3, **dadurch gekennzeichnet, daß** die nicht siliconhaltigen, organischen Monomere, die die Hauptkette des gepfropften Siliconpolymers bilden, unter den auf radikalischem Wege polymerisierbaren Monomeren mit ethylenischer Doppelbindung, den durch Polykondensation polymerisierbaren Monomeren und den unter Ringöffnung polymerisierbaren Monomeren ausgewählt sind.

5. Zusammensetzung nach einem der Ansprüche 1 bis 4, **dadurch gekennzeichnet, daß** sie mindestens ein gepfropftes Siliconcopolymer enthält, das aufweist:
a) 0 bis 98 Gew.-% mindestens eines lipophilen Monomers (A) von geringer Polarität mit ethylenischer Doppelbindung, das radikalisch polymerisierbar ist;
b) 0 bis 98 Gew.-% mindestens eines polaren hydrophilen Monomers (B) mit ethylenischer Doppelbindung, das mit dem (den) Monomer(en) vom Typ (A) copolymerisierbar ist;
c) 0,01 bis 50 Gew.-% mindestens eines Polysiloxanmakromers (C) der allgemeinen Formel:
X(Y)ₙSi(R)₃₋ₘZₘ (I)
worin bedeuten:
- X eine mit den Monomeren (A) und (B) copolymerisierbare Vinylgruppe;
- Y eine zweiwertige Verbindungsgruppe;
- R Wasserstoff, Alkyl oder Alkoxy mit 1 bis 6 Kohlenstoffatomen, C₆₋₁₂-Aryl;
- Z eine einwertige Polysiloxaneinheit mit einem Zahlenmittel des Molekulargewichts von mindestens 500;
- n 0 oder 1 und m eine ganze Zahl von 1 bis 3; wobei die Prozentzahlen bezogen auf das Gesamtgewicht der Monomeren (A), (B) und (C) angegeben sind.

6. Zusammensetzung nach Anspruch 5, **dadurch gekennzeichnet, daß** die lipophilen Monomere (A) unter Estern von Acrylsäure oder Methacrylsäure und C₁₋₁₈-Alkoholen; Styrol; Polystyrolmakromeren; Vinylacetat; Vinylpropionat; α-Methylstyrol; tert.-Butylstyrol; Butadien; Cyclohexadien; Ethylen; Propylen; Vinyltoluol; Estern von Acrylsäure oder Methacrylsäure und 1,1-Dihydroperfluoralkanolen oder ihren homologen Verbindungen; Estern von Acrylsäure oder Methacrylsäure und omega-Hydridofluoralkanolen; Estern von Acrylsäure oder Methacrylsäure und Fluoralkylsulfonamidoalkoholen; Estern von Acrylsäure oder Methacrylsäure und Fluoralkylalkoholen; Estern von Acrylsäure oder Methacrylsäure und Fluorethern von Alkoholen; oder deren Gemischen ausgewählt sind.

7. Zusammensetzung nach Anspruch 6, **dadurch gekennzeichnet, daß** die lipophilen Monomere (A) unter n-Butylmethacrylat, Isobutylmethacrylat, tert.-Butylacrylat, tert.-Butylmethacrylat, 2-Ethylhexylmethacrylat, Methylmethacrylat, 2-(N-Butyl-perfluoroctan-sulfonamido)-ethylacrylat; und 2-(N-Methyl-perfluoroctan-sulfonamido)-ethylacrylat ausgewählt sind.

8. Zusammensetzung nach einem der Ansprüche 5 bis 7, **dadurch gekennzeichnet, daß** die polaren Monomere (B) unter Acrylsäure, Methacrylsäure, N,N-Dimethylacrylamid, Dimethylaminoethylmethacrylat, quaternisiertem Dimethylaminoethylmethacrylat, (Meth)acrylamid, N-t-Butyl-acrylamid, Maleinsäure, Maleinsäureanhydrid und seinen Halbestem, hydroxyalkylierten (Meth)acrylaten, Diallyldimethylammoniumchlorid, Vinylpyrrolidon, Vinylethern, Maleimiden, Vinylpyridin, Vinylimidazol, heterocyclischen polaren Vinylverbindungen, Styrolsulfonat, Allylalkohol, Vinylalkohol, Vinylcaprolactam oder deren Gemischen ausgewählt sind.

9. Zusammensetzung nach Anspruch 8, **dadurch gekennzeichnet, daß** die polaren Monomere (B) unter Acrylsäure, N,N-Dimethylacrylamid, Dimethylaminoethylmethacrylat, quaternisiertem Dimethylaminoethylmethacrylat, Vinylpyrrolidon und deren Gemischen ausgewählt sind.

10. Zusammensetzung nach einem der Ansprüche 5 bis 9, **dadurch gekennzeichnet, daß** das Polysiloxanmakromer (C) der folgenden allgemeinen Formel (II) entspricht: 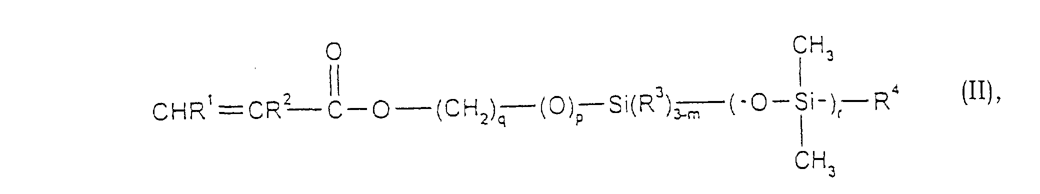 worin bedeuten:
R¹ Wasserstoff oder ―COOH,
R² Wasserstoff, Methyl oder ―CH₂COOH,
R³ Alkyl, Alkoxy oder Alkylamino mit 1 bis 6 Kohlenstoffatomen, C₆₋₁₂-Aryl oder Hydroxy,
R⁴ Alkyl, Alkoxy oder Alkylamino mit 1 bis 6 Kohlenstoffatomen, C₆₋₁₂-Aryl oder Hydroxy,
q eine ganze Zahl von 2 bis 6,
p 0 oder 1,
r eine ganze Zahl von 5 bis 700,
m eine ganze Zahl von 1 bis 3.

11. Zusammensetzung nach einem der Ansprüche 5 bis 10, **dadurch gekennzeichnet, daß** das Polysiloxanmakromer (C) der folgenden allgemeinen Formel entspricht: 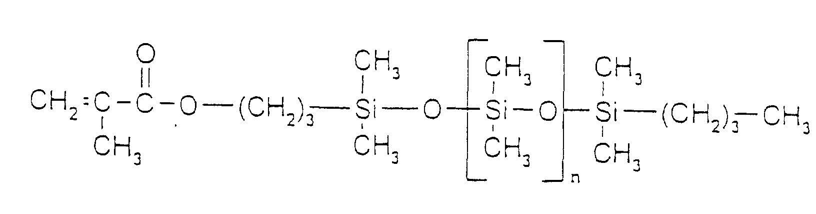 wobei n eine Zahl im Bereich von 5 bis 700 bedeutet.

12. Zusammensetzung nach einem der Ansprüche 1 bis 11, **dadurch gekennzeichnet, daß** sie mindestens ein Copolymer enthält, das durch radikalische Polymerisation ausgehend von folgendem Monomerengemisch erhältlich ist:
a. 60 Gew.-% tert.-Butylacrylat,
b. 20 Gew.-% Acrylsäure,
c. 20 Gew.-% Siliconmakromer der Formel: 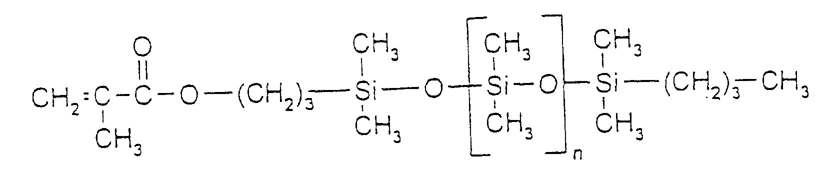
wobei n eine Zahl von 5 bis 700 ist; die Prozentzahlen sind bezogen auf das Gesamtgewicht der Monomere angegeben.

13. Zusammensetzung nach einem der Ansprüche 1 bis 11, **dadurch gekennzeichnet, daß** sie mindestens ein Copolymer enthält, das durch radikalische Polymerisation ausgehend von folgendem Monomerengemisch erhältlich ist:
a) 80 Gew.-% tert.-Butylacrylat,
b) 20 Gew.-% Siliconmakromer der Formel: 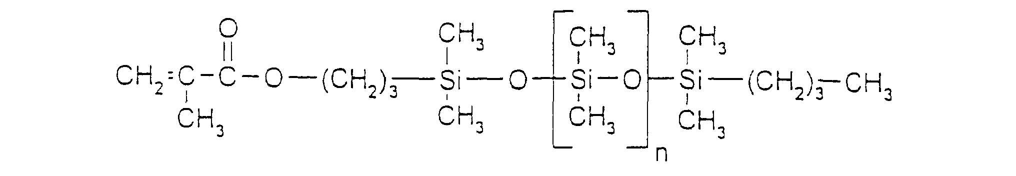
wobei n eine Zahl von 5 bis 700 ist; die Prozentzahlen sind bezogen auf das Gesamtgewicht der Monomere angegeben.

14. Zusammensetzung nach einem der Ansprüche 5 bis 13, **dadurch gekennzeichnet, daß** das Polymer mit einem nicht siliconhaltigen, organischen Grundgerüst, das mit polysiloxanhaltigen Monomeren gepfropft ist, ein Zahlenmittel des Molekulargewichts von 10 000 bis 2 000 000 und eine Glasübergangstemperatur Tg oder eine kristalline Schmelztemperatur Tₘ von mindestens ―20 °C aufweist.

15. Zusammensetzung nach einem der Ansprüche 1 bis 4, **dadurch gekennzeichnet, daß** sie mindestens ein Polymer mit einem nicht siliconhaltigen, organischen Grundgerüst enthält, das mit polysiloxanhaltigen Monomeren gepfropft ist, das durch reaktive Extrusion eines Polysiloxanmakromers mit einer reaktiven Endgruppe und eines Polymers vom Polyolefintyp erhältlich ist, das Gruppen aufweist, die mit der reaktiven Endgruppe des Polysiloxanmakromers reagieren können, wodurch eine kovalente Bindung ausgebildet wird, über die das Silicon auf die Polyolefin-Hauptkette gepfropft werden kann.

16. Zusammensetzung nach Anspruch 15, **dadurch gekennzeichnet, daß** das reaktive Polyolefin unter den Polyethylenen oder den Polymeren von Monomeren, die von Ethylen abgeleitet sind, ausgewählt sind, die reaktive Gruppen aufweisen, die mit der Endgruppe des Polysiloxanmakromers reagieren können.

17. Zusammensetzung nach Anspruch 15 oder 16, **dadurch gekennzeichnet, daß** das reaktive Polyolefin unter den Copolymeren von Ethylen oder Derivaten von Ethylen und Monomeren, die eine Carboxygruppe aufweisen; Monomeren, die eine Anhydridgruppe aufweisen; Monomeren, die ein Säurechloridgruppe enthalten; Monomeren, die eine Estergruppe aufweisen; und Monomeren, die eine Isocyanatgruppe enthalten, ausgewählt sind.

18. Zusammensetzung nach einem der Ansprüche 15 bis 17, **dadurch gekennzeichnet, daß** das Polysiloxanmakromer ein Polysiloxan ist, das am Ende der Polysiloxankette oder in der Nähe des Kettenendes eine funktionelle Gruppe aufweist, die unter Alkoholen, Thiolen, Epoxy und primären und sekundären Aminen ausgewählt ist.

19. Zusammensetzung nach einem der Ansprüche 15 bis 18, **dadurch gekennzeichnet, daß** das Polysiloxanmakromer ein Polysiloxan ist, das der folgenden allgemeinen Formel (III) entspricht:
T-(CH₂)ₛ-Si-[-(OSiR⁵R⁶)ₜ-R⁷]_{y} (III),
worin T unter NH₂, NHR', Epoxy, OH und SH ausgewählt ist; R⁵, R⁶, R⁷ und R' unabhängig voneinander C₁₋₆-Alkyl, Phenyl, Benzyl, C₆₋₁₂-Alkylphenyl oder Wasserstoff bedeuten; s eine Zahl von 2 bis 100; t eine Zahl von 0 bis 1 000; und y eine Zahl von 1 bis 3 ist.

20. Zusammensetzung nach Anspruch 1 oder 2, **dadurch gekennzeichnet, daß** sie mindestens ein gepfropftes Siliconpolymer mit einem Polysiloxangerüst enthält, das mit nicht siliconhaltigen, organischen Monomeren gepfropft ist, die eine Siliconhauptkette enthalten, auf die in der Kette sowie gegebenenfalls an mindestens einem ihrer Enden mindestens eine organische Gruppe gepfropft ist, die kein Silicon enthält.

21. Zusammensetzung nach Anspruch 20, **dadurch gekennzeichnet, daß** das Polymer mit einem Polysiloxangerüst, das mit nicht siliconhaltigen organischen Monomeren gepfropft ist, durch radikalische Copolymerisation mindestens eines nicht siliconhaltigen, anionischen, organischen Monomers, das eine ethylenische Doppelbindung aufweist, und/oder eines nicht siliconhaltigen, hydrophoben, organischen Monomers, das eine ethylenische Doppelbindung aufweist, und eines Polysiloxans, das in seiner Kette mindestens eine funktionelle Gruppe enthält, die mit den ethylenischen Doppelbindungen der nicht siliconhaltigen Monomere reagieren kann, hergestellt werden kann.

22. Zusammensetzung nach Anspruch 21, **dadurch gekennzeichnet, daß** das anionische, organische Monomer mit ethylenischer Doppelbindung einzeln oder in Form von Monomerengemischen unter den geradkettigen oder verzweigten, ungesättigten Carbonsäuren ausgewählt ist.

23. Zusammensetzung nach Anspruch 22, **dadurch gekennzeichnet, daß** das anionische, organische Monomer mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure oder deren Alkalisalzen, Erdalkalisalzen oder Ammoniumsalzen oder deren Gemischen ausgewählt ist.

24. Zusammensetzung nach Anspruch 22, **dadurch gekennzeichnet, daß** das hydrophobe, organische Monomer mit ethylenischer Doppelbindung einzeln oder in Form von Monomerengemischen unter den Estern von Acrylsäure und Alkanolen und/oder Estern von Methacrylsäure und Alkanolen oder deren Gemischen ausgewählt ist, wobei die Alkanole vorzugsweise 1 bis 18 Kohlenstoffatome aufweisen.

25. Zusammensetzung nach Anspruch 24, **dadurch gekennzeichnet, daß** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Methyl(meth)acrylat, tert.-Butyl(meth)acrylat, Tridecyl(meth)acrylat und Stearyl(meth)acrylat ausgewählt ist.

26. Zusammensetzung nach einem der Ansprüche 20 bis 25, **dadurch gekennzeichnet, daß** das gepfropfte Siliconpolymer an der Siliconhauptkette mindestens eine organische Gruppe mit anionischen Eigenschaften enthält, die durch radikalische (Homo)polymerisation mindestens eines anionischen Monomers vom Typ einer ungesättigten, ganz oder teilweise in Form eines Salzes neutralisierten Carbonsäure erhalten wird.

27. Zusammensetzung nach einem der Ansprüche 20 bis 26, **dadurch gekennzeichnet, daß** das gepfropfte Siliconpolymer unter den Siliconpolymeren ausgewählt ist, die in ihrer Struktur die Einheit der folgenden Formel (IV) aufweisen: 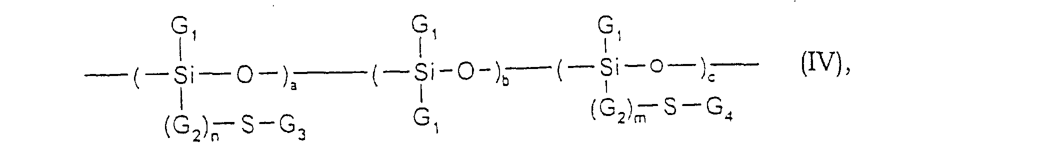 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350; und c 0 oder eine ganze Zahl von 1 bis 50 bedeuten, mit der Maßgabe, daß einer der Parameter a oder c von 0 verschieden ist.

28. Zusammensetzung nach Anspruch 27, **dadurch gekennzeichnet, daß** die Einheit der Formel (IV) mindestens eine der folgenden Eigenschaften aufweist:
- die Gruppen G₁ bedeuten C₁₋₁₀-Alkyl;
- n ist nicht Null und die Gruppen G₂ bedeuten eine zweiwertige Gruppe mit 1 bis 3 Kohlenstoffatomen;
- G₃ bedeutet eine Polymergruppe, die aus der (Homo)polymerisation mindestens eines Monomers vom Typ einer Carbonsäure mit ethylenischer Doppelbindung resultiert;
- G₄ bedeutet eine Polymergruppe, die aus der (Homo)polymerisation mindestens eines Monomers vom Typ C₁₋₁₀-Alkyl(meth)-acrylat resultiert.

29. Zusammensetzung nach Anspruch 27 oder 28, **dadurch gekennzeichnet, daß** die Einheit der Formel (IV) gleichzeitig die folgenden Eigenschaften aufweist:
- die Gruppen G₁ bedeuten Methyl;
- n ist nicht Null und die Gruppen G₂ bedeuten Propylen;
- G₃ bedeutet eine Polymergruppe, die aus der (Homo)polymerisation von zumindest Acrylsäure und/oder Methacrylsäure resultiert;
- G₄ bedeutet eine Polymergruppe, die aus der (Homo)polymerisation von zumindest Isobutyl(meth)acrylat oder Methyl(meth)-acrylat resultiert.

30. Zusammensetzung nach einem der Ansprüche 20 bis 29, **dadurch gekennzeichnet, daß** das Zahlenmittel des Molekulargewichts des gepfropften Polymers mit Polysiloxangerüst im Bereich von etwa 10 000 bis 1 000 000 und noch bevorzugter etwa 10 000 bis 100 000 liegt.

31. Zusammensetzung nach einem der Ansprüche 1 bis 30, **dadurch gekennzeichnet, daß** das gepfropfte Siliconpolymer oder die gepfropften Siliconpolymere in Konzentrationen von 0,01 bis 20 Gew.-%, bezogen auf das Gesamtgewicht der Zusammensetzung, vorzugsweise im Bereich von 0,1 bis 15 Gew.-% und insbesondere im Bereich von 0,5 bis 10 Gew.-% verwendet werden.

32. Zusammensetzung nach einem der Ansprüche 1 bis 31, **dadurch gekennzeichnet, daß** das verdickende Polymer oder Copolymer von (Meth)acrylamid oder eines (Meth)acrylamidderivats unter:
(i) den vernetzten Copolymeren von 2-Acrylamido-2-methylpropansulfonsäure, die ganz oder zum Teil neutralisiert sind und acrylamid;
(ii) den vernetzten oder nicht vernetzten Copolymeren von Ammoniumacrylat und (Meth)acrylamid;
(iii) den vernetzten oder nicht vernetzten Copolymeren von Dimethylaminoethylmethacrylat, das mit Methylchlorid vernetzt ist, und (Meth)acrylamid; und
(iv) deren Gemischen ausgewählt ist.

33. Zusammensetzung nach einem der Ansprüche 1 bis 32, **dadurch gekennzeichnet, daß** das oder die verdickenden Polymere oder Copolymere von (Meth)acrylamid oder eines (Meth)acrylamidderivats in einem Mengenanteil von 0,01 bis 20 Gew.-%, bezogen auf das Gesamtgewicht der Zusammensetzung, vorzugsweise 0,1 bis 15 Gew.-% und noch bevorzugter 0,5 bis 10 Gew.-% verwendet werden.

34. Zusammensetzung nach einem der Ansprüche 1 bis 33, **dadurch gekennzeichnet, daß** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

35. Zusammensetzung nach einem der Ansprüche 1 bis 34, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

36. Zusammensetzung nach Anspruch 35, **dadurch gekennzeichnet, daß** die kosmetisch akzeptablen Lösungsmittel unter Monoalkoholen, Polyalkoholen, Glykolethern, Fettsäureestern und deren Gemischen ausgewählt sind.

37. Zusammensetzung nach einem der Ansprüche 1 bis 36, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

38. Zusammensetzung nach einem der Ansprüche 1 bis 36, **dadurch gekennzeichnet, daß** es sich bei den Keratinsubstanzen um menschliches Haar handelt.

39. Zusammensetzung nach einem der Ansprüche 1 bis 38, **dadurch gekennzeichnet, daß** sie als Gel, Milch, Creme, mehr oder weniger dickflüssige Lotion oder Schaum vorliegt.

40. Zusammensetzung nach einem der Ansprüche 1 bis 39, **dadurch gekennzeichnet, daß** es sich um ein Produkt zum Frisieren handelt.

41. Zusammensetzung nach einem der Ansprüchel bis 40, **dadurch gekennzeichnet, daß** es sich um ein Produkt für das Haar handelt, das unter den Haarwaschmitteln und Produkten für das Haar ausgewählt ist, die ausgespült oder nicht ausgespült werden und vor oder nach einer Haarwäsche, Färbung, Entfärbung, Dauerwelle oder Entkräuselung aufgetragen werden.

42. Zusammensetzung nach einem der Ansprüche 1 bis 41, **dadurch gekennzeichnet, daß** sie in einem Zerstäuber, Pumpflakon oder Aerosolbehälter konfektioniert ist, um ein Spray, einen Lack oder einen Schaum zu erhalten.

43. Nicht therapeutisches Verfahren zur Behandlung von Keratinsubstanzen und insbesondere menschlichem Haar, **dadurch gekennzeichnet, daß** es darin besteht, auf die Keratinsubstanzen eine Zusammensetzung nach einem der Ansprüche 1 bis 42 aufzutragen und gegebenenfalls mit Wasser zu spülen.

## Claims

1. Cosmetic or dermatological composition for treating keratin materials, **characterized in that** it contains, in a cosmetically or dermatologically acceptable medium, at least one grafted silicone polymer comprising a polysiloxane portion and a portion consisting of a non-silicone organic chain, one of the two portions constituting the main chain of the polymer, the other being grafted onto the said main chain and at least one thickening polymer or copolymer of (meth)acrylamide or of a (meth)acrylamide derivative, which may be crosslinked or non-crosslinked.

2. Composition according to Claim 1, **characterized in that** the grafted silicone polymer is chosen from the group consisting of polymers containing a non-silicone organic skeleton grafted with monomers containing a polysiloxane, polymers containing a polysiloxane skeleton grafted with non-silicone organic monomers, and mixtures thereof.

3. Composition according to Claim 1 or 2, **characterized in that** it contains at least one grafted silicone polymer, containing a non-silicone organic skeleton, consisting of an organic main chain formed from organic monomers not comprising silicone, onto which is grafted, within the said chain and also optionally on at least one of its ends, at least one polysiloxane macromer.

4. Composition according to Claim 3, **characterized in that** the non-silicone organic monomers constituting the main chain of the grafted silicone polymer are chosen from the group consisting of free-radical-polymerizable monomers containing ethylenic unsaturation, polycondensation-polymerizable monomers and ring-opening monomers.

5. Composition according to any one of Claims 1 to 4, **characterized in that** it contains at least one grafted silicone copolymer comprising:
a) from 0% to 98% by weight of at least one free-radical-polymerizable lipophilic monomer (A) of low polarity containing ethylenic unsaturation of low polarity;
b) from 0% to 98% by weight of at least one polar hydrophilic monomer (B) containing ethylenic unsaturation, which is copolymerizable with the monomer(s) of the type (A);
c) from 0.01% to 50% by weight of at least one polysiloxane macromer (C) of general formula:
X(Y)ₙSi(R)₃₋ₘZₘ (I)
in which:
X denotes a vinyl group which is copolymerizable with the monomers (A) and (B);
Y denotes a divalent bonding group;
R denotes a hydrogen, a C₁-C₆ alkyl or alkoxy, or a C₆-C₁₂ aryl;
Z denotes a monovalent polysiloxane unit with a number-average molecular weight of at least 500;
n is 0 or 1 and m is an integer ranging from 1 to 3;
the percentages being calculated relative to the total weight of the monomers (A), (B) and (C).

6. Composition according to Claim 5, **characterized in that** the lipophilic monomers (A) are chosen from the group consisting of acrylic or methacrylic acid esters of C₁-C₁₈ alcohols; styrene; polystyrene macromers; vinyl acetate; vinyl propionate; α-methylstyrene; tert-butylstyrene; butadiene; cyclohexadiene; ethylene; propylene; vinyltoluene; acrylic or methacrylic acid esters of a 1,1'-dihydroperfluoroalkanol or homologues thereof; acrylic or methacrylic acid esters of an ω-hydridofluoroalkanol; acrylic or methacrylic acid esters of a fluoroalkylsulphoamidoalcohol; acrylic or methacrylic acid esters of fluoroalkyl alcohols; acrylic or methacrylic acid esters of fluoroether alcohols; or mixtures thereof.

7. Composition according to Claim 6, **characterized in that** the lipophilic monomers (A) are chosen from the group consisting of n-butyl methacrylate, isobutyl methacrylate, tert-butyl acrylate, tert-butyl methacrylate, 2-ethylhexyl methacrylate, methyl methacrylate, 2-(N-methylperfluorooctanesulphonamido)-ethyl acrylate and 2-(butylperfluorooctanesulphonamido)ethyl acrylate.

8. Composition according to any one of Claims 5 to 7, **characterized in that** the polar monomers (B) are chosen from the group consisting of acrylic acid, methacrylic acid, N,N-dimethylacrylamide, dimethylaminoethyl methacrylate, quaternized dimethylaminoethyl methacrylate, (meth)acrylamide, N-t-butylacrylamide, maleic acid, maleic anhydride and hemiesters thereof, hydroxyalkyl (meth)acrylates, diallyldimethylammonium chloride, vinylpyrrolidone, vinyl ethers, maleimides, vinylpyridine, vinylimidazole, heterocyclic vinyl polar compounds, styrene sulphonate, allyl alcohol, vinyl alcohol and vinylcaprolactam, or mixtures thereof.

9. Composition according to Claim 8, **characterized in that** the polar monomers (B) are chosen from the group consisting of acrylic acid, N,N-dimethylacrylamide, dimethylaminoethyl methacrylate, quaternized dimethylaminoethyl methacrylate and vinylpyrrolidone, and mixtures thereof.

10. Composition according to any one of Claims 5 to 9, **characterized in that** the polysiloxane macromer (C) corresponds to the general formula (II) below: 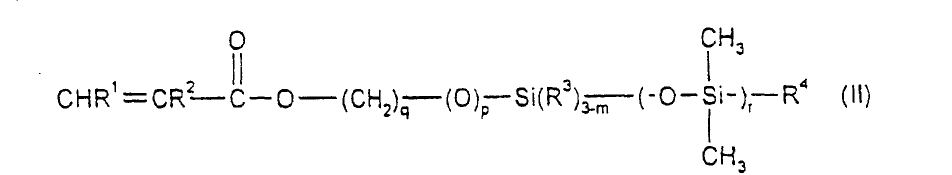 in which:
R¹ is hydrogen or -COOH;
R² is hydrogen, methyl or -CH₂COOH;
R³ is C₁-C₆ alkyl, alkoxy or alkylamino, C₆-C₁₂ aryl or hydroxyl;
R⁴ is C₁-C₆ alkyl, alkoxy or alkylamino, C₆-C₁₂ aryl or hydroxyl;
q is an integer from 2 to 6;
p is 0 or 1;
r is an integer from 5 to 700;
m is an integer from 1 to 3.

11. Composition according to any one of Claims 5 to 10, **characterized in that** the polysiloxane macromer (C) corresponds to the following general formula: 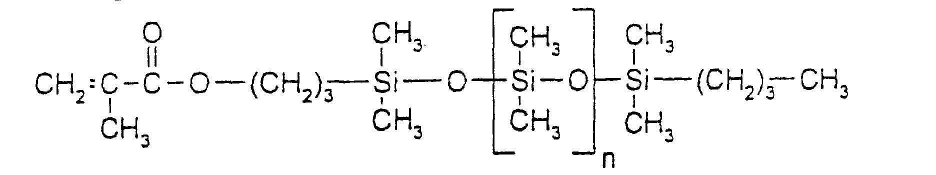 with n being a number ranging from 5 to 700.

12. Composition according to any one of Claims 1 to 11, **characterized in that** it contains at least one copolymer which may be obtained by free-radical polymerization starting with the monomer mixture consisting of:
a) 60% by weight of tert-butyl acrylate;
b) 20% by weight of acrylic acid;
c) 20% by weight of silicone macromer of formula: 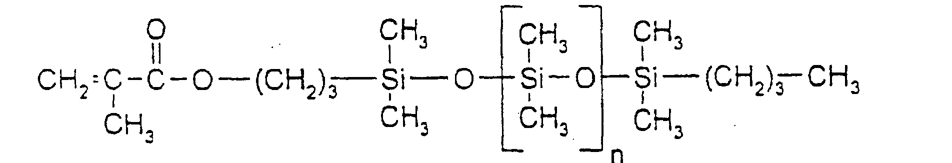 with n being a number ranging from 5 to 700; the weight percentages being calculated relative to the total weight of the monomers.

13. Composition according to any one of Claims 1 to 11, **characterized in that** it contains at least one copolymer which may be obtained by free-radical polymerization starting with the monomer mixture consisting of:
a) 80% by weight of tert-butyl acrylate;
b) 20% by weight of silicone macromer of formula: 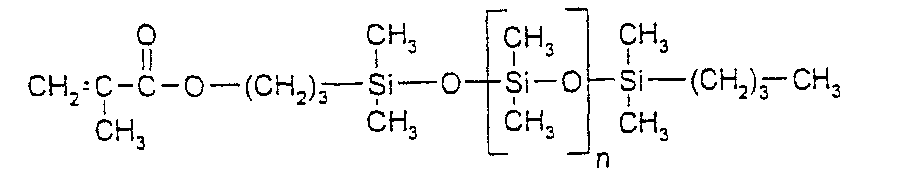 with n being a number ranging from 5 to 700; the weight percentages being calculated relative to the total weight of the monomers.

14. Composition according to any one of Claims 5 to 13, **characterized in that** the polymer containing a non-silicone organic skeleton grafted with monomers containing a polysiloxane has a number-average molecular weight ranging from 10,000 to 2,000,000 and a glass transition temperature Tg or a crystal melting point Tm of at least -20°C.

15. Composition according to any one of Claims 1 to 4, **characterized in that** it contains at least one polymer containing a non-silicone organic skeleton grafted with monomers containing a polysiloxane, which may be obtained by reactive extrusion-moulding of a polysiloxane macromer with a reactive terminal function on a polymer of the polyolefin type comprising reactive groups capable of reacting with the reactive terminal function of the polysiloxane macromer to form a covalent bond for grafting the silicone onto the main chain of the polyolefin.

16. Composition according to Claim 15, **characterized in that** the reactive polyolefin is chosen from the group consisting of polyethylenes and polymers of ethylene-derived monomers comprising reactive functions capable of reacting with the terminal function of the polysiloxane macromer.

17. Composition according to Claim 15 or 16, **characterized in that** the reactive polyolefin is chosen from the group consisting of copolymers of ethylene or of ethylene derivatives and of monomers chosen from those comprising a carboxylic function; those comprising an acid anhydride function; those comprising an acid chloride function; those comprising an ester function; those comprising an isocyanate function.

18. Composition according to any one of Claims 15 to 17, **characterized in that** the polysiloxane macromer is a polysiloxane comprising a functionalized group, at the end of the polysiloxane chain or close to the end of the said chain, chosen from the group consisting of alcohols, thiols, epoxy groups and primary and secondary amines.

19. Composition according to any one of Claims 15 to 18, **characterized in that** the polysiloxane macromer is a polysiloxane corresponding to the general formula (III):
T-(CH₂)ₛ-Si-[-(OSiR⁵R⁶)ₜ-R⁷]_{y} (III)
in which T is chosen from the group consisting of NH₂, NHR' and an epoxy, OH or SH function; R⁵, R⁶, R⁷ and R' independently denoting a C₁-C₆ alkyl, phenyl, benzyl, C₆-C₁₂ alkylphenyl or hydrogen; s is a number from 2 to 100; t is a number from 0 to 1000 and y is a number from 1 to 3.

20. Composition according to Claim 1 or 2, **characterized in that** it contains at least one grafted silicone polymer, containing a polysiloxane skeleton grafted with non-silicone organic monomers, comprising a polysiloxane main chain onto which is grafted, within the said chain and also optionally on at least one of its ends, at least one organic group not comprising silicone.

21. Composition according to Claim 20, **characterized in that** the polymer containing a polysiloxane skeleton grafted with non-silicone organic monomers may be obtained by free-radical copolymerization between, on the one hand, at least one non-silicone anionic organic monomer containing ethylenic unsaturation and/or a non-silicone hydrophobic organic monomer containing ethylenic unsaturation, and, on the other hand, a polysiloxane containing in its chain at least one functional group capable of reacting with the said ethylenic unsaturations of the said non-silicone monomers.

22. Composition according to Claim 21, **characterized in that** the anionic organic monomer containing ethylenic unsaturation is chosen, alone or in the form of a monomer mixture, from linear or branched unsaturated carboxylic acids.

23. Composition according to Claim 22, **characterized in that** the anionic organic monomer containing ethylenic unsaturation is chosen, alone or in the form of a monomer mixture, from acrylic acid, methacrylic acid, maleic acid, maleic anhydride, itaconic acid, fumaric acid and crotonic acid or alkali metal salts, alkaline-earth metal salts or ammonium salts thereof, or mixtures thereof.

24. Composition according to Claim 22, **characterized in that** the hydrophobic organic monomer containing ethylenic unsaturation is chosen, alone or as a monomer mixture, from acrylic acid esters of an alkanol and/or methacrylic acid esters of an alkanol, the alkanol preferably being C₁-C₁₈.

25. Composition according to Claim 24, **characterized in that** the hydrophobic organic monomer containing ethylenic unsaturation is chosen, alone or as a monomer mixture, from the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

26. Composition according to any one of Claims 20 to 25, **characterized in that** the grafted silicone polymer comprises on the main silicone chain at least one organic group of anionic nature obtained by free-radical (homo)polymerization of at least one anionic monomer of unsaturated carboxylic acid type, which is partially or totally neutralized in the form of a salt.

27. Composition according to any one of Claims 20 to 26, **characterized in that** the grafted silicone polymer is chosen from silicone polymers comprising in their structure the unit of formula (IV) below: 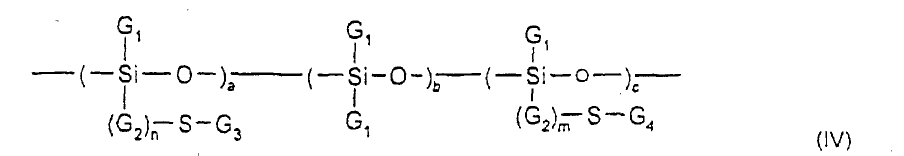 in which the radicals G₁, which may be identical or different, represent hydrogen, a C₁-C₁₀ alkyl radical or a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350; c is an integer ranging from 0 to 50; with the proviso that one of the parameters a to c is other than 0.

28. Composition according to Claim 27, **characterized in that** the unit of formula (IV) has at least one of the following characteristics:
- the radicals G₁ denote a C₁-C₁₀ alkyl radical;
- n is non-zero and the radicals G₂ represent a divalent C₁-C₃ radical;
- G₃ represents a polymer radical resulting from the (homo)polymerization of at least one monomer of the carboxylic acid type containing ethylenic unsaturation;
- G₄ represents a polymer radical resulting from the (homo)polymerization of at least one monomer of the (C₁-C₁₀) alkyl (meth) acrylate type.

29. Composition according to Claim 27 or 28, **characterized in that** the unit of formula (IV) simultaneously has the following characteristics:
- the radicals G₁ denote a methyl radical;
- n is non-zero and the radicals G₂ denote a propylene radical;
- G₃ represents a polymeric radical resulting from the (homo)polymerization of at least acrylic acid and/or methacrylic acid;
- G₄ represents a polymeric radical resulting from the (homo)polymerization of at least one monomer such as isobutyl or methyl (meth)acrylate.

30. Composition according to any one of Claims 20 to 29, **characterized in that** the number-average molecular mass of the polymer containing a polysiloxane skeleton grafted with non-silicone organic monomers ranges from 10,000 to 1,000,000 approximately and even more preferably from 10,000 to 100,000 approximately.

31. Composition according to any one of Claims 1 to 30, **characterized in that** the grafted silicone polymer(s) is(are) used in an amount ranging from 0.01% to 20% by weight relative to the total weight of the composition, preferably from 0.1% to 15% by weight and even more preferably from 0.5% to 10% by weight.

32. Composition according to any one of Claims 1 to 31, **characterized in that** the thickening polymer or copolymer of (meth)acrylamide or of a (meth)acrylamide derivative is chosen from the group consisting of:
(i) partially or totally neutralized crosslinked copolymers of 2-acrylamido-2-methylpropanesulphonic acid and of acrylamide;
(ii) crosslinked or non-crosslinked copolymers of ammonium acrylate and of (meth)acrylamide;
(iii) crosslinked or non-crosslinked copolymers of dimethylaminoethyl methacrylate quaternized with methyl chloride and of (meth)acrylamide;
(iv) mixtures thereof.

33. Composition according to any one of Claims 1 to 32, **characterized in that** the thickening polymers or copolymers of (meth)acrylamide or of a (meth)acrylamide derivative is(are) used in an amount ranging from 0.01% to 20% by weight relative to the total weight of the composition, more preferably from 0.1% to 15% by weight and even more preferably from 0.5% to 10% by weight.

34. Composition according to any one of Claims 1 to 33, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils and any other additive conventionally used in cosmetics.

35. Composition according to any one of Claims 1 to 34, **characterized in that** the cosmetically or dermatologically acceptable medium consists of water or a mixture of water and at least one cosmetically acceptable solvent.

36. Composition according to Claim 35, **characterized in that** the cosmetically acceptable solvents are chosen from the group consisting of monoalcohols, polyalcohols, glycol ethers and fatty acid esters, and mixtures thereof.

37. Composition according to any one of Claims 1 to 36, **characterized in that** the grafted silicone polymer is dissolved in the cosmetically or dermatologically acceptable medium or is used in the form of an aqueous dispersion of particles.

38. Composition according to any one of Claims 1 to 36, **characterized in that** the keratin materials are human hair.

39. Composition according to any one of Claims 1 to 38, **characterized in that** it is in the form of a gel, a milk, a cream, a more or less thickened lotion or a mousse.

40. Composition according to any one of Claims 1 to 39, **characterized in that** it is a hairstyling product.

41. Composition according to any one of Claims 1 to 40, **characterized in that** it is a haircare product chosen from the group consisting of shampoos and rinse-out or leave-in hair products to be applied before or after shampooing, dyeing, bleaching, permanent-waving or straightening the hair.

42. Composition according to any one of Claims 1 to 41, **characterized in that** it is packaged in the form of a vaporizer or a pump-dispenser bottle or in an aerosol container in order to obtain a spray, a lacquer or a mousse.

43. Non-therapeutic process for treating keratin materials, in particular human hair, **characterized in that** it consists in applying a composition as defined according to any one of Claims 1 to 42 to the said materials and then in optionally rinsing it out with water.
